# EUROPÄISCHE PATENTANMELDUNG

(11) **EP 0 962 465 A1**
(43) Veröffentlichungstag der Anmeldung: **08.12.1999**
(21) Anmeldenummer: 99109732.0
(22) Anmeldetag: 18.05.1999
(51) Int. Cl.: C07K 7/02, C07K 7/06, C07K 7/56, C07K 7/64, C07K 1/10

(54) **Verfahren zur Cyclisierung von Penta- und Hexapeptiden**

(30) Priorität: 30.05.1998 DE 19824449
(71) Anmelder: Clariant GmbH, 65929 Frankfurt am Main (DE)
(72) Erfinder: Mollenkopf, Carl Christoph, Dr., 65929 Frankfurt (DE); Henklein,, Peter, Dr., 10365 Berlin (DE)

(57) **Zusammenfassung**

Offenkettige Penta- und Hexapeptide, insbesondere H₂N-Ile-Leu-Asp(O-tBu)-Val-NH(CH₂)₅-COOH und H₂H-Phe-D-Trp-Ala-Thr(tBu)-Phe-Pro-COOH, werden mit Hilfe von (PrPO₂)₃ (= Propanphosphonsäureanhydrid) quantitativ zu den entsprechenden Cyclopeptiden cyclisiert.

## Beschreibung

Es sind zahlreiche Cyclopenta- und Cyclohexapeptide bekannt, die in der Medizin für therapeutische Zwecke eingesetzt werden. Die Herstellung dieser Peptide erfolgt üblicherweise so, daß z.B. in einem Peptidsynthesizer zunächst die offenkettige Form des gewünschten Penta- oder Hexapeptids hergestellt wird, die anschließend mit Hilfe eines Kondensationsmittels cyclisiert wird.

So wird beispielsweise von R. Hirschmann, J. Med. Chem. 1996, 39, 2441 - 2448 beschrieben, daß der cyclische Somatostatin-Agonist L-363,301 durch Cyclisierung von H₂N-Phe-D-Trp-Ala-Thr(tBu)-Phe-Pro-COOH mit Hilfe von Phosphorsäurediphenylesterazid (DPPA) in einer Ausbeute von 82 % hergestellt wird.

Weiterhin ist in WO 96/20 216 eine Cyclisierung von H₂N-Ile-Leu-Asp(O^{t}Bu)-Val-NH(CH₂)₅-COOH mit 2-(1H-Benzotriazol-1-yl)-1,1,3,3-tetramethyluroniumhexafluorophosphat (= HBTU) in einer Ausbeute von 67,7 % beschrieben.

Es bestand daher die Aufgabe, ein Verfahren zu entwickeln, mit dem in einfacher Weise die Cyclisierung von Penta- und Hexapeptiden in hoher Reinheit und in deutlich besseren Ausbeuten gelingt.

Es wurde gefunden, daß die Aufgabe überraschenderweise gelöst wird, wenn als Cyclisierungsmittel Propanphosphonsäureanhydrid (= T₃P) verwendet wird.

Gegenstand der Erfindung ist ein Verfahren zur Cyclisierung von Penta- oder Hexapeptiden, dadurch gekennzeichnet, daß das offenkettige Penta- oder Hexapeptid mit Propanphosphonsäureanhydrid umgesetzt wird.

Propanphosphonsäureanhydrid, (PrPO₂)₃, ist dem Fachmann bekannt und kommerziell erhältlich.
Die erfindungsgemäße Umsetzung wird zweckmäßig in einem organischen Lösungsmittel wie Dimethylformamid, CH₂Cl₂, N-Methylpyrrolidon oder Essigsäureethylester durchgeführt.

Weiterhin vorteilhaft ist die Gegenwart basischer Verbindungen, insbesondere tertiärer Amine, wie Diisopropylethylamin, Triethylamin, Ethylmorpholin, Methylmorpholin, Pyridin oder Collidin.

Die Umsetzung wird normalerweise bei Temperaturen von -10 bis +30°C, vorzugsweise +5 bis 25°C, durchgeführt. Die Reaktionszeit kann 2 bis 60 Stunden betragen.

Es ist vorteilhaft, wenn Propanphosphonsäureanhydrid (T₃P) im 3 bis 7-fachen, vorzugsweise 4 bis 5-fachen, molaren Überschuß, bezogen auf das Peptid, eingesetzt wird. Die basische Verbindung wird zweckmäßig im Verhältnis 0,8:1 bis 1,5:1, vorzugsweise in etwa equimolaren Mengen, bezogen auf T₃P, eingesetzt.

Nach Beendigung der Reaktion wird das Lösungsmittel entfernt, z.B. durch Abdestillieren im Hochvakuum, das Peptid üblicherweise in Wasser gelöst und lyophilisiert.

Die erfindungsgemäße Umsetzung ist besonders vorteilhaft für die Cyclisierung von H₂N-Phe-D-Trp-Ala-Thr(tBu)-Phe-Pro-COOH und H₂N-Ile-Leu-Asp(O-tBu)-Val-NH(CH₂)₅-COOH, wobei ein vollständiger Umsatz erzielt wird.

In den nachfolgenden Beispielen werden folgende Abkürzungen verwendet:
- T₃P =: Propanphosphonsäureanhydrid, (PrPO₂)₃;
- Fmoc =: 9-Fluorenyl-methoxycarbonyl;
- TBTU =: 2-(1H-Benzotriazol-1-yl)-1,1,3,3-tetramethyluronium-tetrafluoroborat;
- HOBT =: 1-Hydroxy-benzotriazol;
- TCP =: Tritylchlorid-Polystyrol-Harz;
- DIPEA =: Diisopropylethylamin;
- DMF =: Dimethylformamid.

### Beispiel 1

### H₂N-Phe-D-Trp-Ala-Thr(tBu)-Phe-Pro-COOH (H2)

Das offenkettige Peptid H2 wurde an einem 433 A Peptid Synthesizer der Firma Applied Biosystems synthetisiert. Als Trägerharz diente TCP-Harz-L-Pro-Fmoc (PepChem, Tübingen) mit einer Beladung von 0,62 mmol/g.
0,5 g (0,31 mmol) Harz wurden mit einem 3,1-fachen Überschuß der jeweiligen Aminosäure durch Doppelkopplung beladen. Als Kopplungsreagenz wurde TBTU mit HOBT und DIPEA verwendet. Die Abspaltung des fertigen Peptids vom Harz erfolgte mit Essigsäure (50 %), Methanol (10 %) und Dichlormethan (40 %) in 1,5 Stunden. Nach Entfernung der Abspaltreagenzien im Vakuum wurde das Peptid aus Diethylether gefällt, abgesaugt, in Wasser gelöst und lyophilisiert.
- Verwendete Aminosäuren:: Fmoc-Phe-OH
Fmoc-Thr (t-Bu)-OH
Fmoc-L-Ala-OH
Fmoc-D-Trp-OH
Fmoc-Phe-OH

### Cyclisierung:

0,0192 g (0,025 mmol) H2 wurden in 25 ml DMF gelöst. Zu der Lösung wurden 0,026 ml (0,15 mmol) DIPEA und 0,079 ml (0,125 mmol) T₃P (50 %ig in DMF) gegeben. Die Reaktionszeit betrug 48 h bei Raumtemperatur. Anschließend wurde das Lösungsmittel im Hochvakuum entfernt, das Peptid in Wasser gelöst und lyophilisiert. Die Cyclisierung zu c(Phe-D-Trp-Ala-Thr(tBu)-Phe-Pro-CO) verlief vollständig, bezogen auf Ausgangspeptid.

### Beispiel 2

### H₂N-Ile-Leu-Asp(O^{t}Bu)-Val-NH(CH₂)₅-COOH (H4)

0,5 g (0,725 mmol) TCP-Harz wurden mit 0,384 g (1,087 mmol) Fmoc-6-aminocapronsäure beladen. Dazu wurde die Aminosäure in 4 ml Dichlormethan gelöst, 0,2 ml DIPEA hinzugegeben und die gesamte Lösung zum trockenen Harz gegeben. Nach 5 Minuten Rühren wurde das restliche DIPEA (insgesamt 3,26 mmol) zugesetzt. Die Reaktionszeit betrug 1 h. Danach wurden 0,4 ml Methanol hinzugegeben und 20 min gerührt.
- Das Harz wurde abfiltriert und gewaschen:: 3 x Dichlormethan
2 x DMF
3 x Methanol

Nach der Reinigung wurde es im Exsikkator getrocknet.

Das offenkettige Peptid H4 wurde an einem 433 A Peptid Synthesizer der Firma Applied Biosystems synthetisiert. Als Trägerharz diente das Fmoc-6-aminocapronsäure-TCP-Harz (PepChem, Tübingen) mit einer Beladung von 1,45 mmol/g.
0,25 g (0,362 mmol) Harz wurden mit einem 1,4-fachen Überschuß der jeweiligen Aminosäure durch Doppelkopplung beladen. Als Kopplungsreagenz wurde TBTU mit HOBT und DIPEA verwendet. Die Abspaltung des fertigen Peptids vom Harz erfolgte mit Essigsäure (50 %), Methanol (10 %) und Dichlormethan (40 %) in 1,5 Stunden. Nach Entfernung der Abspaltreagenzien im Vakuum wurde das Peptid aus Diethylether gefällt, abgesaugt, in Wasser gelöst und lyophilisiert.
- Verwendete Aminosäuren:: Fmoc-Val-OH
Fmoc-Asp (Ot-Bu)-OH
Fmoc-Leu-OH
Fmoc-Ile-OH

### Cyclisierung:

0,01 g (0,0159 mmol) H4 wurden in 16 ml DMF gelöst. Zu der Lösung wurden 0,0166 ml (0,095 mmol) DIPEA und 0,051 ml (0,08 mmol) T₃P (50 %ig in DMF) gegeben. Die Reaktionszeit betrug 48 h bei Raumtemperatur. Anschließend wurde das Lösungsmittel im Hochvakuum entfernt, das Peptid in Wasser gelöst und lyophilisiert. Die Cyclisierung zu c(Ile-Leu-Asp(O-tBu)-Val-NH(CH₂)₅-CO) verlief vollständig.

## Patentansprüche

1. Verfahren zur Cyclisierung von Penta- oder Hexapeptiden, dadurch gekennzeichnet, daß das offenkettige Penta- oder Hexapeptid mit Propanphosphonsäureanhydrid umgesetzt wird.

2. Verfahren nach Anspruch 1, dadurch gekennzeichnet, daß das offenkettige Pentapeptid H₂N-Ile-Leu-Asp(O-tBu)-Val-NH(CH₂)₅-COOH ist.

3. Verfahren nach Anspruch 1, dadurch gekennzeichnet, daß das offenkettige Hexapeptid H₂N-Phe-D-Trp-Ala-Thr(tBu)-Phe-Pro-COOH ist.

4. Verfahren nach mindestens einem der Ansprüche 1 bis 3, dadurch gekennzeichnet, daß die Umsetzung in einem organischen Lösungsmittel durchgeführt wird.

5. Verfahren nach mindestens einem der Ansprüche 1 bis 4, dadurch gekennzeichnet, daß die Umsetzung in Dimethylformamid, Dichlormethan, Essigsäureethylester oder N-Methylpyrrolidon durchgeführt wird.

6. Verfahren nach mindestens einem der Ansprüche 1 bis 5, dadurch gekennzeichnet, daß die Umsetzung in Gegenwart einer basischen Verbindung durchgeführt wird.

7. Verfahren nach mindestens einem der Ansprüche 1 bis 6, dadurch gekennzeichnet, daß die Umsetzung in Gegenwart von Diisopropylethylamin, Triethylamin, Pyridin, N-Methylmorpholin, N-Ethylmorpholin oder Collidin durchgeführt wird.

8. Verfahren nach mindestens einem der Ansprüche 1 bis 7, dadurch gekennzeichnet, daß die Umsetzung bei einer Temperatur von -10 bis +30°C, vorzugsweise von +5 bis 25°C, durchgeführt wird.

9. Verfahren nach mindestens einem der Ansprüche 1 bis 8, dadurch gekennzeichnet, daß Propanphosphonsäureanhydrid in einem 3 bis 7-fachen molaren Überschuß, bezogen auf das Peptid, eingesetzt wird.

10. Verfahren nach mindestens einem der Ansprüche 1 bis 9, dadurch gekennzeichnet, daß die basische Verbindung im Verhältnis 0,8:1 bis 1,5:1, bezogen auf Propanphosphonsäureanhydrid, eingesetzt wird.
